(19) Europäisches Patentamt
European Patent Office
Office européen des brevets

(11) Publication number: **0 244 097**
**A2**

# (12) EUROPEAN PATENT APPLICATION

(21) Application number: 87302917.7

(22) Date of filing: 03.04.87

(51) Int. Cl.³: C 07 D 487/04
A 01 N 43/90
//(C07D487/04, 239:00, 231:00)

(30) Priority: 30.04.86 GB 8610531

(43) Date of publication of application:
04.11.87 Bulletin 87/45

(84) Designated Contracting States:
AT BE CH DE ES FR GB GR IT LI LU NL SE

(71) Applicant: SCHERING AGROCHEMICALS LIMITED
Hauxton Cambridge CB2 5HU(GB)

(72) Inventor: Hunt, Russell George
of 45 The Limes
Harston Cambridge(GB)

(74) Representative: Wells, Norman David et al,
Schering Agrochemicals Limited Industrial Property
Department Chesterford Park Research Station
Saffron Walden Essex CB10 1XL(GB)

## (54) Pyrazolopyrimidine herbicides.

(57) The invention provides certain herbicidal pyrazolopyrimidine derivatives, processes for their preparation, and compositions containing them, the compounds being of the formula:

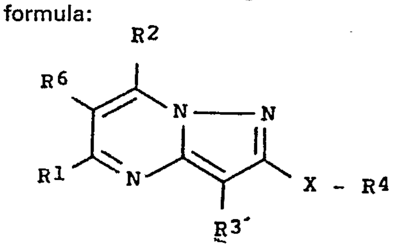

(I)

and salts thereof, where:

$R^1$ and $R^2$, which may be the same or different, each represent hydrogen, hydroxy, halo, substituted or unsubstituted alkyl, alkoxy, alkylthio, alkenyl, alkynyl or phenyl, or heterocyclyl;

$R^6$ represents hydrogen, halo, cyano, carboxy, alkoxycarbonyl, or substituted or unsubstituted alkyl or carbamoyl; or $R^6$ and one of $R^1$ and $R^2$ together represent an alkylene chain of 3 or 4 carbon atoms:

$R^3$ represents hydrogen, alkyl, aryl, aralkyl, carboxy, substituted or unsubstituted alkoxycarbonyl, carbamoyl or thiocarbamoyl, cyano or nitro;

X represents $-NR-SO_2-$, $-SO_2-O-$, $-SO_2-NR-$ or $-S(O)_n-CR'R''-$, where n is 0, 1 or 2 and R, R' and R″ each represent hydrogen, or substituted or unsubstituted alkyl, alkenyl, alkynyl, alkoxycarbonyl, acyl, alkylsulphonyl or arylsulphonyl; and

$R^4$ represents hydrogen, heterocyclyl, or a substituted or unsubstituted alkyl or aryl group.

EP 0 244 097 A2

Case 86/10531

Pyrazolopyrimidine herbicides

This invention concerns herbicidal pyrazolopyrimidine derivatives, processes for their preparation, and compositions containing them.

In one aspect, the invention provides the pyrazolopyrimidine derivatives of the formula:

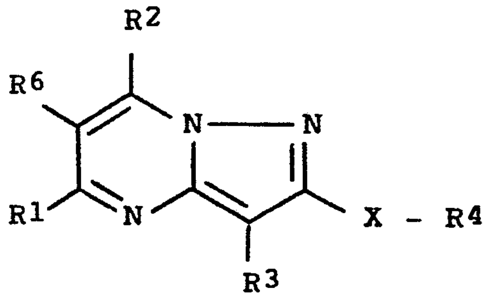

(I)

and salts thereof, where:

$R^1$ and $R^2$, which may be the same or different, each represent hydrogen, hydroxy, halo, substituted or unsubstituted alkyl, alkoxy, alkylthio, alkenyl, alkynyl or phenyl, or heterocyclyl;

$R^6$ represents hydrogen, halo, cyano, carboxy, alkoxycarbonyl, or sustituted or unsubstituted alkyl or carbamoyl; or $R^6$ and one of $R^1$ and $R^2$ together represent an alkylene chain of 3 or 4 carbon atoms;

$R^3$ represents hydrogen, alkyl, aryl, aralkyl, carboxy, substituted or unsubstituted alkoxycarbonyl, carbamoyl or thiocarbamoyl, cyano or nitro;

X represents $-NR-SO_2-$, $-SO_2-O-$, $-SO_2-NR-$ or $-S(O)_n-CR'R''-$, where n is 0, 1 or 2 and R, R' and R"

each represent hydrogen, or substituted or unsubstituted alkyl, alkenyl, alkynyl, alkoxycarbonyl, acyl, alkylsulphonyl or arylsulphonyl; and

$R^4$ represents hydrogen, heterocyclyl, or a substituted or unsubstituted alkyl or aryl group.

When $R^1$, $R^2$ or $R^6$ represents halo, it is preferably chloro or bromo.

When $R^1$ or $R^2$ represents or contains an alkyl group, that group is preferably of 1 to 6 carbon atoms, especially of 1 to 4 carbon atoms. Specific preferred unsubstituted alkyl or alkyl-containing groups which $R^1$, and $R^2$ may represent include methyl, ethyl, n-propyl, isopropyl, n-butyl, t-butyl, methoxy, ethoxy, n-propoxy, methylthio and ethylthio. The alkyl group may if desired be substituted, for example by one or more halogen atoms, eg fluorine, chlorine or bromine, or by alkoxy groups of 1 to 4 carbon atoms, eg methoxy or ethoxy. Specific preferred sustituted alkyl-containing groups which $R^1$, and $R^2$ may represent include chloromethyl, bromomethyl, dichloromethyl, trifluoromethyl, difluoromethoxy, difluoromethylthio, methoxyethyl and ethoxyethyl.

When $R^1$ or $R^2$ represents an alkenyl or alkynyl group, that group is preferably of 2 to 6 carbon atoms, for example allyl, vinyl or propargyl. Any such alkenyl or alkynyl group is preferably unsubstituted.

When $R^1$ or $R^2$ represents a phenyl group, it is preferably substituted, for example by one or more halogen atoms, eg fluorine, chlorine or bromine, nitro groups, substituted or unsubstituted amino groups (eg alkylamino, dialkylamino or acylamino groups, especially where the alkyl moiety has from 1 to 4 carbon atoms), cyano groups, or alkyl or alkoxy groups of 1 to 4 carbon atoms, eg methyl, ethyl, methoxy or ethoxy.

When $R^1$, $R^2$ or $R^4$ represents a heterocyclic group, that group is preferably a monocyclic group of 5 or 6 ring atoms which contains at least one atom of nitrogen, oxygen or sulphur. Preferred heterocyclic groups include pyridinyl, pyrrolyl, piperidinyl, morpholinyl and thienyl groups.

$R^1$ and $R^2$ independently preferably represent hydrogen, hydroxy, methyl, trifluoromethyl or chloro.

When $R^6$ represents or contains an alkyl group, that group is preferably of 1 to 6 carbon atoms, especially of 1 to 4 carbon atoms. Specific preferred groups which $R^6$ may represent include methyl, ethyl, n-propyl, isopropyl, n-butyl, t-butyl, carboxy, methoxycarbonyl, ethoxycarbonyl, carbamoyl, methylcarbamoyl and dimethylcarbamoyl.

$R^6$ preferably represents hydrogen, chloro or methyl, or together with $R^2$ represents a trimethylene chain.

When $R^3$ represents or contains an alkyl group, that group is preferably of 1 to 6 carbon atoms, especially of 1 to 4 carbon atoms. Specific preferred groups containing unsubstituted alkyl moieties which $R^3$ may represent include methyl, ethyl, n-propyl, isopropyl, n-butyl, t-butyl, methoxycarbonyl, ethoxycarbonyl, isopropoxycarbonyl, n-butoxycarbonyl, methylcarbamoyl and dimethylcarbamoyl. The alkyl group may if desired be substituted, for example by one or more halogen atoms, eg fluorine, chlorine or bromine, or by alkoxy groups of 1 to 4 carbon atoms, eg methoxy or ethoxy. Specific preferred groups containing substituted alkyl moieties which $R^3$ may represent include chloromethyl, bromomethyl, dichloromethyl, trifluoromethyl and ethoxyethoxycarbonyl.

When $R^3$ represents or contains an aryl or aralkyl group, it preferably represents or contains a phenyl or benzyl group, which is desirably substituted by one or more alkyl or alkoxy groups of 1 to 4 carbon atoms, halogen atoms, nitro groups or substituted or unsubstituted amino groups (eg alkylamino or dialkylamino groups, especially where the alkyl moiety is of 1 to 4 carbon atoms).

$R^3$ preferably represents carboxy, substituted or unsubstituted alkoxycarbonyl of 2 to 5 carbon atoms (for example methoxycarbonyl or ethoxycarbonyl), carbamoyl or

substituted carbamoyl (for example hydrazinocarbonyl or alkyl-substituted hydrazinocarbonyl).

When $R^4$ represents an alkyl group, that group is preferably of 1 to 6 carbon atoms, especially of 1 to 4 carbon atoms. Specific preferred unsubstituted alkyl groups which $R^4$ may represent include methyl, ethyl, n-propyl, isopropyl, n-butyl and t-butyl. The alkyl group may if desired be substituted, for example by one or more halogen atoms, eg fluorine, chlorine or bromine, or by alkoxy groups of 1 to 4 carbon atoms, eg methoxy or ethoxy. Specific preferred substituted alkyl groups which $R^4$ may represent include chloromethyl, bromomethyl, dichloromethyl, trifluoromethyl, methoxyethyl and ethoxyethyl.

$R^4$ preferably represents a phenyl group substituted by one or more chlorine atoms, methyl groups, alkoxycarbonyl groups (especially of 2 to 5 carbon atoms), nitro groups or substituted or unsubstituted amino groups (especially alkylamino or dialkylamino groups in which the alkyl moieties are of 1 to 4 carbon atoms).

When R, R' or R" represents or contains alkyl, that moiety is preferably of 1 to 6 carbon atoms, especially of 1 to 4 carbon atoms. Specific preferred groups which R, R'and R" may represent include methyl, ethyl, n-propyl, isopropyl, n-butyl, t-butyl, acetyl, methoxycarbonylmethyl, propanoyl, methoxycarbonyl, ethoxycarbonyl, methylsulphonyl, ethylsulphonyl and benzenesulphonyl.

When R, R' or R" represents alkenyl or alkynyl, it is preferably of 2 to 6 carbon atoms, for example vinyl, allyl or propargyl.

X preferably represents $-SO_2-NH-$.

Preferred salts of the compounds of formula I are those of the compounds where X represents $-SO_2-NR-$, particularly alkali-metal salts, eg sodium or potassium salts, ammonium salts, trialkylammonium salts, and salts with heterocyclic bases, eg the pyridinium salts.

In a particularly preferred group of compounds of formula I, one of $R^1$ and $R^2$ represents methyl and the other represents hydrogen or methyl, $R^6$ is hydrogen, $R^3$ is methoxycarbonyl, ethoxycarbonyl, carbamoyl, methylcarbamoyl or dimethylcarbamoyl, X is $-SO_2NH-$, and $R^4$ is phenyl substituted by one or more fluorine, chlorine or bromine atoms, alkoxycarbonyl groups of 2 to 5 carbon atoms, or methyl or nitro groups.

Especially preferred compounds according to the invention are those of the Examples provided hereinafter though particular mention may be made of methyl 2-(2,6-dichlorophenylsulphamoyl)-5,7-dimethylpyrazolo-[1,5-a]pyrimidine-3-carboxylate and methyl 2-(2,6-dichlorophenylsulphamoyl)-5-methyl-7-trifluoromethylpyrazolo-[1,5-a]pyrimidine-3-carboxylate.

In another aspect, the invention provides a process for the preparation of a pyrazolopyrimidine derivative of formula I by reacting an aminopyrazole of the formula:

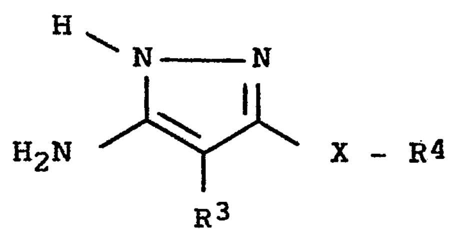

(II)

where $R^3$, $R^4$, R and R' are as defined hereinbefore, in a suitable solvent medium, with a diketone of the formula:

$$R^1COCHR^6COR^2 \quad (III)$$

where $R^1$, $R^2$ and $R^6$ are as defined hereinbefore, to give the desired compound.

The solvent medium employed is preferably an alkanol such as ethanol, or acetic acid, and the reaction is desirably carried out with heating, eg to reflux.

The compounds of formula I in which X represents $-SO_2-NR-$ or $-SO_2-O-$ may be prepared by a process in which a pyrazolopyrimidine sulphonyl halide of the formula:

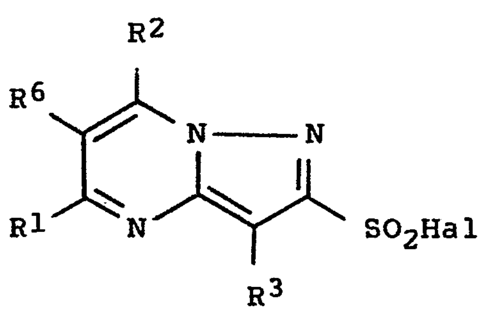

(IV)

where $R^1$, $R^2$, $R^3$ and $R^6$ are as defined hereinbefore, and Hal represents halogen, is reacted in the presence of a base with a compound of the formula $R^4NHR$ or $R^4OH$ where R and $R^4$ are as defined hereinbefore to give the desired compound where X is respectively $-SO_2NR-$ or $-SO_2O-$.

The reaction is conveniently effected at room temperature or with heating, eg from 10 to 100°C, and in a suitable solvent medium, for example pyridine.

The base is preferably an organic base, especially a tertiary orgaic base, for example 4-dimethylaminopyridine.

The compounds of formula IV may themselves be prepared by reacting a compound of formula I wherein $X-R^4$ represents benzylthio with the appropriate halogen or sulphuryl halide in a suitable solvent medium, to give the desired compound.

The reaction is desirably effected with cooling to less than ambient temperature, eg to a temperature of -10°C to 5°C.

The compounds of formula I where X represents $-NR-SO_2-$ may alternatively be prepared by reaction of a pyrazolopyrimidine amine of the formula:

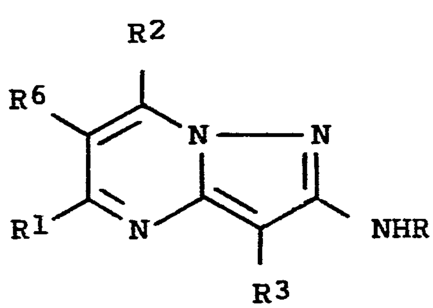

(V)

where $R^1$, $R^2$, $R^3$, $R^6$ and R are as defined hereinbefore in the presence of a base with a sulphonyl halide of the formula $R^4SO_2Hal$ where $R^4$ is as defined herinbefore and Hal represents halogen to give the desired compound.

In turn, the pyrazolopyrimidine amines of formula V may be prepared by a process in which a diaminopyrazole of the formula:

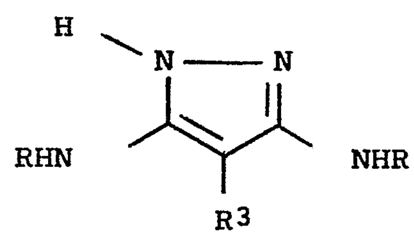

(VI)

where each R is the same and is as defined hereinbefore and $R^3$ is as defined hereinbefore is reacted with a diketone of the formula $R^1COCHR^6COR^2$ where $R^1$, $R^2$ and $R^6$ are as defined hereinbefore to give the desired compound.

The reaction is desirably effected in a suitable solvent medium and in the presence of a base catalyst.

The solvent employed may for example be an alkanol such as ethanol, and the base catalyst employed is suitably an amine, eg triethylamine.

The diamines of formula VI are either known compounds or may be prepared by methods known per se.

Salts of the compounds of formula I may be prepared by reaction thereof with a suitable base containing the desired cation, eg the appropriate trialkylamine.

The compounds of formula I may also be interconverted by methods known per se. For example, the compounds of formula I in which X represents a group $-SO_2NR$ where R is other than hydrogen, may in general be prepared from the corresponding compounds of formula I where R is hydrogen by reaction thereof in the presence of a strong base, for example sodium hydride, with a halide of formula RHal.

The compounds of formula I where X represents $-S(O)_n-CRR'-$ where n is 1 or 2 may be prepared from the corresponding compounds of formula I where n is 0 by selective oxidation techniques known per se.

Carboxy groups present in the compounds of formula I may be esterified, ester groups present may be hydrolysed to give the corresponding acids, and cyano or ester groups may be converted to amido or substituted amido groups. Such transformations, as well as many others, may be effected by techniques well-known to those skilled in the art, and various examples of such transformations are provided hereinafter.

The compounds of formula I are herbicidally-active against a wide range of broad-leaved and grassy weeds, but

are comparatively safe to certain crop species. They may thus be of use as selective herbicides, particularly in the control of a range of weeds in cereals or other crops, eg wheat, barley, maize, soya beans, oilseed rape, cotton or sugar beet.

In another aspect, the invention provides a herbicidal composition which comprises one or more compounds of the invention in association with a suitable carrier and/or surface active agent.

The compositions usually contain from 0.01 to 99% by weight of the present compounds, and are normally produced initially as concentrates containing from 0.5 to 99%, preferably from 0.5 to 85%, and especially from 10 to 50% by weight thereof. Such concentrates are diluted if necessary before application to the locus to be treated such that the active ingredient comprises from 0.01 to 5% by weight of the formulation applied.

The carrier may be water, in which case an organic solvent may also be present, though this is not usually employed. A flowable suspension concentrate may be formed by grinding the compound with water, a wetting agent and a suspending agent, e.g. xanthan gum.

The carrier may alternatively be a water immiscible organic solvent, e.g. a hydrocarbon which boils within the range 130-270°C, e.g. xylene, in which the compound is

dissolved or suspended. An emulsifiable concentrate containing a water immiscible solvent may be formed with a surface active agent so that the concentrate acts as a self-emulsifiable oil on admixture with water.

The carrier may alternatively be a water-miscible organic solvent e.g. 2-methoxy ethanol, methanol, propylene glycol, diethylene glycol, diethylene glycol monoethyl ether, methylformamide or dimethylformamide.

The carrier may alternatively be a solid, which may be finely divided or granular. Examples of suitable solids are limestone, clays, sand, mica, chalk, attapulgite, diatomite, perlite, sepiolite, silicas, silicates, lignosulphonates and solid fertilizers. The carrier can be of natural or synthetic origin or can be modified natural material.

Wettable powders soluble or dispersible in water may be formed by admixing the compound in particulate form with a particulate carrier or spraying molten compound on to the particulate carrier, admixing a wetting agent and a dispersing agent and finely grinding the whole powder mixture.

An aerosol composition may be formed by admixing the compound with a propellant, e.g. a polyhalogenated alkane such as dichlorofluoromethane, and suitably also with a solvent.

The term 'surface active agent' is used in the broad sense to include materials variously called emulsifying agents, dispersing agents and wetting agents. Such agents are well known in the art.

The surface active agents used may comprise anionic surface active agents, for example mono- or di-esters of phosphoric acid with a fatty alcohol ethoxylate, or salts of such esters, fatty alcohol sulphates such as sodium dodecyl sulphate, ethoxylated fatty alcohol sulphates, ethoxylated alkylphenol sulphates, lignin sulphates, petroleum sulphonates, alkylaryl sulphonates such as alkyl-benzene sulphonates or lower alkylnaphthalene sulphonates, salts of sulphonated naphthaleneformaldehyde condensates, salts of sulphonated phenolformaldehyde condensates, or more complex sulphonates such as the amide sulphonates, e.g. the sulphonated condensation product of oleic acid and N-methyl taurine or the dialkyl sulphosuccinates e.g. the sodium sulphonate of dioctyl succinate.

The surface active agents may also comprise non-ionic agents, for example condensation products or fatty acid esters, fatty alcohols, fatty acid amides or alkyl-substituted phenols with ethylene oxide, fatty esters of polyhydric alcohol ethers e.g. sorbitan fatty acid esters, condensation products of such esters with

ethylene oxide e.g. polyoxyethylene sorbitan fatty acid esters, block copolymers of ethylene oxide and propylene oxide, acetylenic glycols such as 2,4,7,9-tetramethyl-5-decyn-4,7-diol, or ethoxylated acetylenic glycols.

The surface active agents may also comprise cationic agents, for example alkyl- and/or aryl-substituted quaternary ammonium compounds such as cetyl trimethylammonium bromide, or ethoxylated tertiary fatty amines.

Preferred surface active agents include ethoxylated fatty alcohol sulphates, lignin sulphonates, alkyl-aryl sulphonates, salts of sulphonated naphthaleneformaldehyde condensates, salts of sulphonated phenolformaldehyde condensates, sodium oleoyl N-methyltauride, dialkyl sulphosuccinates, alkyl phenol ethoxylates, and fatty alkyl ethoxylates.

The present active compounds, especially those of the Examples provided hereinafter, and particularly methyl 2-(2,6-dichlorophenylsulphamoyl)-5,7-dimethylpyrazolo-[1,5-a]pyrimidine-3-carboxylate and methyl 2-(2,6-dichlorophenylsulphamoyl)-5-methyl-7-trifluoromethylpyrazolo-[1,5-a]pyrimidine-3-carboxylate, may be admixed with another pesticide, eg a herbicide, fungicide or insecticide, or a plant growth regulator, particularly another herbicide.  Suitable further herbicides include

trietazine, linuron, MCPA, dichlorprop, isoxaben, diflufenican, metolachlor, fluometuron, oxyfluorfen, fomesafen, bentazone, prometryne, norflurazon, chlomazone, EPTC, imazaquin, and especially isoproturon, methabenzthiazuron, trifluralin, ioxynil, bromoxynil, benazolin, mecoprop, fluroxypyr, alachlor, acifluorfen, lactofen, metribuzin and pendimethalin.

The present compounds may be applied to plants, the soil, land or aquatic areas, and particularly to a locus at which a crop is growing or is about to grow. The compounds are active both pre- and post-emergence.

The invention is illustrated by the following Examples, in which Me = methyl, Et = ethyl, Ph = phenyl and cyhex = cyclohexyl.

Preparative Example A

Methyl 2-benzylthio-5,7-dimethylpyrazolo[1,5-a]pyrimidine--3-carboxylate (Compound A1)

(a) Methyl 3,3-di(benzylthio)-2-cyanoacrylate

Sodium (51.8g) was dissolved in methanol (820 ml), and 375 ml of this solution were treated with methyl cyanoacetate (123.8g). The temperature fell from 20°C to about 15°C during the addition. Carbon disulphide (37.5 ml) was added, the temperature rising to about 40°C. The mixture was cooled to 20°C and was treated with a further 188 ml of the methoxide solution followed by carbon

disulphide (19 ml). After cooling again to 20°C, the remaining methoxide solution was added, followed by carbon disulphide (9 ml) at 20°C. The total of carbon disulphide added was 65.5 ml. When the additions were complete, the mixture was stirred at room temperature for 3 hours. Potassium iodide (3g) was added followed dropwise by benzyl chloride (275.8g). The temperature rose to about 55°C during the addition. The mixture was then boiled under reflux for 1 hour. After cooling, the mixture was poured into 5 l of iced water, and the solid material was filtered off, washed thoroughly with water and dried, to give 320.2g of desired product.

(b) <u>Methyl 3-amino-5-benzylthiopyrazole-4-carboxylate</u>

A stirred mixture of the product of stage (a) (87.0g) and hydrazine hydrate (12.3g) in methanol (340 ml) was boiled under reflux for 5 hours. After cooling, the mixture was evaporated to dryness. The residue was stirred with sodium carbonate solution for 15 minutes. The solid was filtered off, washed with water and recrystallised from acetonitrile to give 28.9g of desired product, mp 103-106°C.

(c) <u>Methyl 2-benzylthio-5,7-dimethylpyrazolo[1,5-a] -pyrimidine-3-carboxylate</u>

A stirred mixture of the product of stage (b) (54.7g) and pentane-2,4-dione (20.8g) in glacial acetic acid (425

ml) was boiled under reflux for 3 hours. After standing at room temperature overnight, the solid was filtered off and recrystallised from acetonitrile to give 54.4g of desired product, mp 161-164°C.

Examples A2-A28

The following compounds of formula I where $R^6$ is hydrogen, and X represents $-SCH_2-$ were prepared by methods analogous to that of Example A, but using appropriately protected ketones of formula III where necessary:

| No. | R1 | R2 | R3 | R4 (Ph subst:) | M Pt (°C) |
|---|---|---|---|---|---|
| A2 | Me | Me | COOEt | - | 156-159 |
| A3 | Me | Me | CN | 2-Cl | 165-168 |
| A4 | Me | Me | CN | 2,6-diCl | 206-208 |
| A5 | Me | Me | CN | 2,5-diMe | 177-179 |
| A6 | $CF_3$ | $CF_3$ | CN | - | 137-139 |
| A7 | H ←--→ | Me | CN | - | 112-114 |
| A8 | OH | OH | COOMe | - | 192-194 |
| A9 | Me | OH | COOMe | - | 256-258 |
| A10 | Me | Cl | COOMe | - | 188-190 |
| A11 | Me | Me | COOH | - | 198-199 |
| A12 | H | H | COOMe | - | 169-171 |
| A13 | Cl | Cl | COOMe | - | 208-210 |
| A14 | H | Me | COOMe | - | 180-182 |
| A15 | H | Ph | COOMe | - | 162-164 |

| No. | R1 | R2 | R3 | R4 (Ph subst:) | M Pt (°C) |
|---|---|---|---|---|---|
| A16 | Me | Ph | COOMe | - | 175-177 |
| A17 | Me | $CF_3$ | COOMe | - | 162-164 |
| A18 | Me | Me | $CONMe_2$ | - | 148-150 |
| A19 | $CF_3$ | $CF_3$ | COOMe | - | 118-120 |
| A20 | H | Me | CN | - | 142-144 |
| A21 | Me | Me | CN | - | 138-140 |
| A22 | Me | Me | $CONH_2$ | 4-Cl | 230-232 |
| A23 | Me | Me | $CONH_2$ | - | 242-244 |
| A24 | Me | $CF_3$ | CN | - | 131-133 |

The following compounds of formula I where $R^4$ represents phenyl and X represents $-SCH_2-$ were also prepared by methods analogous to that of Example A:

| No. | R1 | R2 | R6 | R3 | M Pt (°C) |
|---|---|---|---|---|---|
| A25 | Me | Me | Cl | COOMe | 183-185 |
| A26 | Me | Me | Me | COOMe | 183-185 |
| A27 | Me | $-(CH_2)_3-$ | | COOMe | 201-203 |
| A28 | Me | $-(CH_2)_3-$ | | CN | 135-137 |

Preparative Example B

2-(2-Chlorobenzylsulphinyl)-5,7-dimethylpyrazolo[1,5-a]-pyrimidine-3-carbonitrile (Compound B1)

A stirred mixture of the product of Example A3 (20g), dichloromethane (270ml), water (140ml) and concentrated hydrochloric acid (17.7ml) was cooled to about -3°C and treated dropwise with a solution of sodium hypochlorite

(144 ml, 11-12% available chlorine) in water (144 ml) over 20 minutes, keeping the temperature below 0°C. After stirring at 0-5°C for 45 minutes, the mixture was allowed to return to room temperature, and the layers were separated. The aqueous phase was extracted with more dichloromethane. The organic portions were combined , washed with aqueous sodium metabisulphite solution and dried over magnesium sulphate. Filtration and evaporation gave a solid residue which was triturated with hexane and filtered off. The solid material was washed with hot acetone and recrystallised from acetonitrile to give the desired compound, mp 209-211°C.

Preparative Example C

Methyl 2-(benzylsulphonyl)-5,7-dimethylpyrazolo[1,5-a]-pyrimidine-3-carboxylate (Compound C1)

A mixture of the product of Example A(c) (3.9g) and m-chloroperoxybenzoic acid (about 90%, 4.6g) in toluene (50ml) was boiled under reflux for 2 hours. After standing at room temperature overnight, the mixture was added to sodium carbonate solution and extracted with two portions of dichloromethane which were combined, washed with sodium carbonate solution followed by ferrous sulphate solution, and dried over magnesium sulphate. Filtration and evaporation gave a residue which was recrystallised from ethanol to give the desired compound, mp 185-188°C.

Examples C2-C3

The following compounds of formula I where $R^6$ represents hydrogen, and X represents $-SO_2CH_2-$ were prepared by methods analogous to that of Example C:

| No | R1 | R2 | R3 | R4 (Ph subst:) | M Pt (°C) |
|---|---|---|---|---|---|
| C2 | Me | Me | CN | 2-Cl | 215-218 |
| C3 | Me | Me | CN | 2,6-diCl | 236-239 |

Preparative Example D

Methyl 2-(2,6-dichlorophenylsulphamoyl)-5,7-dimethyl-pyrazolo[1,5-a]pyrimidine-3-carboxylate (Compound D1)

(a) Methyl 2-(chlorosulphonyl)-5,7-dimethylpyrazolo[1,5-a]-pyrimidine-3-carboxylate

A vigorously stirred suspension of the product of Example 1c (20g) in a 50/50 mixture of glacial acetic acid and water (116 ml) was cooled to below 0°C and was treated over 2 hours with chlorine whilst maintaining the temperature at 0-2°C. The solid material was then filtered off, washed with water followed by petroleum ether (60-80) and dried in vacuo to give 17.6g of desired product.

(b) Methyl 2-(2,6-dichlorophenylsulphamoyl)-5,7-dimethyl-pyrazolo[1,5-a]pyrimidine-3-carboxylate

A stirred solution of 2,6-dichloroaniline (10.66g) and 4-(dimethylamino)pyridine (0.4g) in dry pyridine (100 ml) was treated portionwise with the crude product of stage (a) (10.0g). A yellow precipitate appeared

immediately following the addition. The suspension was stirred overnight at room temperature, and the precipitate redissolved. The mixture was then evaporated to dryness, the residue being dissolved in dichloromethane, washed with two portions of dilute hydrochloric acid, and dried over magnesium sulphate. Filtration and evaporation gave a residue which was triturated with methanol. The solid product was filtered off and recrystallised from acetonitrile to give 5.5g of the desired compound, mp 250-252°C.

Examples D2-D54

The following compounds of formula I where $R^6$ represents hydrogen, and X represents $-SO_2NH-$ were prepared by methods analogous to that of Example D:

| No. | R1 | R2 | R3 | R4 (Ph subst:) | M Pt (°C) |
|---|---|---|---|---|---|
| D2 | Me | Me | COOMe | 2,6-diCl,3-Me | 218-220 |
| D3 | Me | Me | COOMe | 2-Cl,6-Me | 234-236 |
| D4 | Me | Me | COOMe | $2-CF_3$ | 188-190 |
| D5 | Me | Me | COOMe | 2,6-diF | 196-198 |
| D6 | Me | Me | COOMe | $2-NO_2$ | 138-140 |
| D7 | Me | Me | COOH | 2,6-diCl | 263-265 |
| D8 | Me | Me | CN | 2-Cl | 176-179 |
| D9 | Me | Me | CN | 2,6-diCl | 262-265 |
| D10 | $CF_3$ | $CF_3$ | CN | 2-Cl | 153-156 |
| D11 | Me | H | COOMe | 2,6-diCl,3-Me | 286-289 |

| | | | | | |
|---|---|---|---|---|---|
| D12 | Me | H | COOMe | 2,6-diCl | 244-246 |
| D13 | Me | H | COOMe | 2,6-diF | 197-199 |
| D14 | Me | Me | COOEt | 2,6-diCl | 206-208 |
| D15 | Me | Me | COOiPr | 2,6-diCl | 190-192 |
| D16 | Me | Me | COOMe | 2-Cl,6-F | 182-184 |
| D17 | Me | H | COOH | 2,6-diCl | 244-246 |
| D18 | H | Me | COOMe | 2,6-diCl | 225-227 |
| D19 | Me | H | COOEt | 2,6-diCl | 194-196 |
| D20 | Me | Me | COOnBu | 2,6-diCl | 191-193 |
| D21 | H | Me | COOMe | 2,6-diBr | 209-210 |
| D22 | H | Me | COOMe | 2-Cl,6-Me | 187-189 |
| D23 | H | Me | COOMe | 2,6-diF | 195-197 |
| D24 | H | Me | COOMe | 2,6-diCl,3-Me | 229-231 |
| D25 | Me | Me | COOMe | 2-Me,6-$NO_2$ | 218-220 |
| D26 | H | Ph | COOMe | 2,6-diCl | 254-256 |
| D27 | H | Ph | COOMe | 2,6-diCl,3-Me | 250-252 |
| D28 | Me | Ph | COOMe | 2,6-diCl | 258-261 |
| D29 | Me | $CF_3$ | COOMe | 2,6-diCl | 227-229 |
| D30 | Me | $CF_3$ | COOMe | 2,6-diCl,3-Me | 269-271 |
| D31 | Me | Me | $CONMe_2$ | 2,6-diCl | 233-235 |
| D32 | Me | Me | COOMe | 2,6-diBr | 185-187 |
| D33 | Me | Me | COOMe | 2-Me,6-COOEt | 175-177 |
| D34 | H | Me | CN | 2,6-diCl | 244-246 |
| D35 | Me | Me | CN | 2,6-diF | 249-252 |
| D36 | Me | Me | CN | 2,6-diCl,3-Me | 287-290 |

| | | | | | |
|---|---|---|---|---|---|
| D37 | Me | Me | COOMe | 2,6-diCl-3,5-di$NO_2$ | 250-252 |
| D38 | Me | Me | CN | 2-Cl,6-Me | 255-257 |
| D39 | Me | Me | COOMe | 2-Cl,6-SMe | 220-227 |
| D40 | Me | Me | COOMe | 2-$CHF_2O$ | 196-198 |
| D41 | $CF_3$ | $CF_3$ | COOMe | 2,6-diCl | 172-174 |
| D42 | H | 2-Py | COOMe | 2,6-diCl | 268-270 |
| D43 | Me | Me | COOMe | 4-Cl | 195-198 |
| D44 | Me | Me | COOMe | 2-Cl | 152-154 |
| D45 | Me | Me | COOMe | 2-MeO | 173-177 |
| D46 | Me | Me | COOMe | 2-Me,6-COOMe | 195-198 |
| D47 | Me | Me | CN | 2-Cl,6-F | 268-270 |

The following compound where $R^4$ represents 2-methoxycarbonyl-4-methylthien-3-yl, $R^6$ represents hydrogen, and X represents $-SO_2NH-$ was also prepared by a method analogous to that of Example D:

| No. | R1 | R2 | R3 | M Pt (°C) |
|---|---|---|---|---|
| D48 | Me | Me | COOMe | 233-235 |

The following compounds where X represents $-SO_2NH-$ were also prepared by methods analogous to that of Example D:

| No. | R1 | R2 | R6 | R3 | R4 (Ph subst:) | M.Pt °C |
|---|---|---|---|---|---|---|
| D49 | Me | Me | Me | COOMe | 2,6-diCl | 221-223 |
| D50 | Me | Me | Cl | COOMe | 2,6-diCl,3-Me | 158-160 |
| D51 | Me | Me | Cl | COOMe | 2,6-diCl | 168-171 |
| D52 | Me | Me | Me | COOH | 2,6-diCl | 280-282 |
| D53 | Me | $-(CH_2)_3-$ | | COOMe | 2,6-diCl | 201-203 |
| D54 | Me | $-(CH_2)_3-$ | | CN | 2,6-diCl | 235-237 |

Preparative Example E

2-Chloro-N-(3-cyano-5,7-dimethylpyrazolo[1,5-a]pyrimidin-2-yl)benzenesulphonamide (Compound E1)

(a) N2-(1-amino-2,2-dicyanovinyl)benzohydrazide

A stirred mixture of potassium tricyanomethanide (69.7g), benzohydrazide (68.0g) and water (350 ml) was treated with concentrated hydrochloric acid (45 ml) and the mixture was heated to boiling. A very thick precipitate quickly formed, and the mixture was boiled gently for 5 hours. After standing overnight, the solid was filtered off, washed with water and air-dried to give 100.3g of desired product, mp 182-186°C.

(b) 3,5-Diaminopyrazole-4-carbonitrile

A mixture of the product of stage (a) and 1M sodium hydroxide solution (750 ml) was stirred at room temperature for 5 hours. It was then filtered and the filtrate was acidified with concentrated hydrochloric acid. The precipitated solid was filtered off and washed with water. The filtrate was washed with three portions of dichloromethane, made alkaline with potassium carbonate, and evaporated to dryness. The residue was extracted thoroughly with acetone, which was evaporated to dryness to give a yellow solid material in a yield of 20.3g, mp 139-142°C.

(c) 2-Amino-5,7-dimethylpyrazolo[1,5-a]pyrimidine-3-carbonitrile

A stirred mixture of the product of stage (b) (20.2g), 2,4-pentanedione (18.5g) and triethylamine (15 drops) in ethanol (375 ml) was boiled under reflux for 5 hours. After cooling, the solid material was filtered off and the filtrate was evaporated to dryness. The residue was recrystallised from acetonitrile along with the filtered solid to yield 6.1g of desired product.

(d) 2-Chloro-N-(3-cyano-5,7-dimethylpyrazolo[1,5-a]-pyrimidin-2-yl)benzenesulphonamide

A mixture of the amine product of stage (c) (2.81g), 2-chlorobenzenesulphonyl chloride (3.17g) and a catalytic amount of 4-dimethylaminopyridine in dry pyridine (35 ml) was stirred at room temperature for two days. It was then poured into water and extracted with three portions of dichloromethane which were combined and dried over magnesium sulphate. Filtration and evaporation gave a residue which was recrystallised from acetonitrile. It was then recrystallised a second time from methanol to give 1.3g of desired product, mp 205-208°C.

Examples E2-E4

The following compound of formula I where $R^6$ represents hydrogen was prepared by a method analogous to that of Example E:

| No. | R1 | R2 | R3 | R4 (Ph subst:) | M.Pt (°C) |
|---|---|---|---|---|---|
| E2 | Me | Me | CN | 2-COOMe | 182-185 |
| E3 | Me | Me | COOMe | 2-Cl | 240-242 |
| E4 | Me | Me | COOMe | 2-COOMe | 197-200 |

Preparative Example F

Methyl 5,7-dimethyl-2-(2-methylphenoxysulphonyl)-pyrazolo[1,5-a]pyrimidine-3-carboxylate

A stirred solution of the product of Example D(a) (2.0 g) and o-cresol (0.71 g) in dry pyridine (30 ml) was heated at about 60°C for 3 hours. After standing at room temperature for 2 days, the mixture was poured into water and extracted with 3 portions of dichloromethane which were combined, washed twice with dilute hydrochloric acidand dried over magnesium sulphate. Filtration and evaporation gave a residue which was recrystallised from methanol to give 1.1 g of desired product, mp 112-114°C.

Preparative Example G

Methyl 2-(2,6-dichlorophenyl-N-propargylsulphamoyl)-5,7-dimethyl-pyrazolo[1,5-a]pyrimidine-3-carboxylate (Compound G1)

Sodium hydride (0.3g) was added to a solution of the product of Example D (Compound D1) (4.3g) in dimethylformamide (50 ml), and the mixture was stirred at room temperature for 10 minutes. Propargyl bromide (1.1ml) was then added, and the mixture was refluxed for

5½ hours, then left to cool. It was then poured into 750ml of distilled water, and the precipitate which formed was filtered off and dried. On recrystallisation from ethyl methyl ketone and then from acetonitrile, 1.3g of desired product, mp 225-229°C, was obtained.

Examples G2-G3

The N-allyl derivative (Compound G2), mp 157-160°C, and the N-methyl derivative (Compound G3), mp 202-204°C, corresponding to that of Example G were prepared by methods analogous to those described therein.

Preparative Example H

2-(2,6-Dichlorophenylsulphamoyl)-5,7-dimethylpyrazolo-[1,5-a]pyrimidine-3-carboxamide (Compound H1)

The product of Example D9 (4.6g) was treated with concentrated sulphuric acid and was stirred at room temperature overnight. It was then poured into ice-water, and the precipitated solid was filtered off, washed with water and dried. It was then purified by dissolving it in dimethylformamide, filtering whilst hot, and reprecipitating the product from the filtrate by the addition of water, to give 3.0g of desired product, mp>320°C.

Examples H2-H7

The following compounds of formula I where $R^6$ represents hydrogen were prepared by methods analogous to that of Example H:

| No. | R1 | R2 | R3 | R4 (Ph subst:) | M.Pt (°C) |
|---|---|---|---|---|---|
| H2 | H | Me | $CONH_2$ | 2,6-diCl | 238-240 |
| H3 | Me | Me | $CONH_2$ | 2,6-diCl,3-Me | >320 |
| H4 | Me | Me | $CONH_2$ | 2,6-diF | 233-235 |
| H5 | Me | Me | $CONH_2$ | 2-Cl,6-Me | 255-257 |
| H6 | Me | Me | $CONH_2$ | 2-Cl,6-F | 320 |

The following compound of formula I where $R^2$ and $R^6$ together represent $-(CH_2)_3-$ was prepared by methods analogous to that of Example H:

| No. | R1 | R3 | R4 (Ph subst:) | M.Pt (°C) |
|---|---|---|---|---|
| H7 | Me | $CONH_2$ | 2,6-diCl | 242-244 |

## Preparative Example I

## 2-(2,6-Dichlorophenylsulphamoyl)-N,5,7-trimethylpyrazolo-[1,5-a]pyrimidine-3-carboxamide (Compound I1)

A suspension of the product of Example D1 (4g) in 33% methylamine solution in industrial methylated spirits (65 ml) was heated in a small autoclave at about 100°C overnight. After cooling, the resultant mixture was filtered, the filtered solid being washed with methanol, dried and recrystallised from glacial acetic acid, to give 1.6g of desired product, mp 284-286°C.

## Example I2

The following compound of formula I where $R^6$ represents hydrogen was prepared by a method analogous to that of Example I:

| No. | R1 | R2 | R3 | R4 (Ph subst:) | M.Pt (°C) |
|---|---|---|---|---|---|
| I2 | Me | Me | CONHcyhex | 2,6-diCl | 236-238 |

Preparative Example J

2-Ethoxyethyl 2-(2,6-dichlorophenylsulphamoyl)-5,7-dimethylpyrazolo[1,5-a]pyrimidine-3-carboxylate

A suspension of the product of Example D1 (1.0g) in 2-ethoxyethanol (25ml) was treated with titanium(IV)-butoxide (0.34g) and was refluxed overnight. After cooling, the mixture was poured into dilute hydrochloric acid and extracted with three portions of dichloromethane. These were combined, washed with sodium hydrogen carbonate solution and dried over magnesium sulphate. Filtration and evaporation gave a residue to which toluene was added. The solid material was filtered off, washed with toluene and dried to give 0.8g of desired product, mp 198-200°C.

Preparative Example K

2-(2,6-Dichlorophenylsulphamoyl)-5,7-dimethylpyrazolo-[1,5-a]pyrimidine-3-carbohydrazide

Hydrazine hydrate (0.56ml) was added to a suspension of the product of Example D1 in ethanol (50ml). The mixture was refluxed for 5 days, left to cool to room temperature, and then evaporated to give 4.0g of desired product as a yellow solid, m.p 285-287°C.

Preparative Example L

Isopropyl 2-(2,6-dichlorophenylsulphamoyl)-5,7-dimethyl -pyrazolo[1,5-a]pyrimidine-3-carboxylate

The product of Example D7 (0.8g) in trifluoroacetic anhydride (4ml) was treated with trifluoroacetic acid (2ml), and the resultant solution was treated carefully with propan-2-ol (20ml) and refluxed for 1 hour. The mixture was then evaporated to dryness, the residue was mixed with water, and the solid material was filtered off and recrystallised from propan-2-ol to give 0.6g of the desired product, mp 190-192°C, identical to that of Example D15.

Preparative Example M

Methyl 2-(2,6-dichlorophenylsulphamoyl)-5-hydroxy-7-methyl -pyrazolo[1,5-a]pyrimidine-3-carboxylate (Compound M1)

(a) Methyl 3-acetamido-5-benzylthiopyrazole-4-carboxylate

The product of Example A(b) (50g), acetic anhydride (20g) and acetic acid (100g) were refluxed with stirring for 6 hours. The solvent was then removed, and the residue was recrystallised from toluene to give 49.5g of desired product.

(b) Methyl 5-acetamido-3-chlorosulphonyl-1H-pyrazole-4-carboxylate

The product of stage (a) (20g) was suspended in

concentrated hydrochloric acid and was cooled to -5°C. Acetic acid (15ml) was then added, and chlorine gas was bubbled through with stirring and cooling to maintain the temperature between -5°C and 0°C. A further 10ml of concentrated hydrochloric acid was then added, and after 70 minutes, the solid was filtered off and washed with water/petrol (60-80) to give 15g of desired product.

(c) Methyl 5-acetamido-3-(2,6-dichlorophenylsulphamoyl)-1H-pyrazole-4-carboxylate

2,6-dichloroaniline (5g) and N,N-dimethyl-4-aminopyridine (0.3g) were dissolved in pyridine (50ml), and the product of stage (a) (5g) was added in portions. The mixture was then stirred at room temperature for 24 hours, after which the pyridine was removed, and the mixture was taken into dichloromethane. It was then washed with water, dried, and the solvent was removed. The residue was recrystallised from acetonitrile to give 3.6g of desired product.

(d) Methyl 5-amino-3-(2,6-dichlorophenylsulphamoyl)-1H-pyrazole-4-carboxylate

The product of stage (c) (10g) was dissolved in methanol (50ml) and potassium carbonate (2g) was added. The reaction mixture was then refluxed for 6 hours, allowed to cool, and was poured into 1500ml water. The mixture was acidified with hydrochloric acid to precipitate the desired product in a yield of 5.4g.

(e) Methyl 2-(2,6-dichlorophenylsulphamoyl)-5-hydroxy-7-methyl-pyrazolo[1,5-a]pyrimidine-3-carboxylate

The product of stage (d) (7g) was refluxed overnight with methyl acetoacetate (3g) in trifluoroacetic acid (25ml). The solvent was then removed and the residue was recrystallised from acetonitrile to give 5.6g of desired product, mp 310-312°C.

Example M2

The compound corresponding to that of Example M where the substituent at the 7-position is hydroxy was prepared by an analogous method, mp 264-267°C.

Preparative Example N

Methyl 2-(2,6-dichlorophenylsulphamoyl)-5-chloro-7-methyl-pyrazolo[1,5-a]pyrimidine-3-carboxylate

The product of Example M (4g) was dissolved in pyridine (5ml), and thionyl chloride (25ml) was added. The reaction mixture was then heated to reflux for 4 hours, the solvent was removed, and the residue was purified by chromatography to give 3.6g of the desired product, mp 256-257°C.

Formulation Example

A 10% suspension concentrate of compound D1 was prepared from the following ingredients:

| | g/1 |
|---|---|
| Compound D1 | 100 |
| Pluronic P75 | 30 |
| Polyfon H | 20 |
| Propylene glycol | 105 |
| Anti-foam 1520 | 0.5 |
| Veegum R | 7.5 |
| Kelzan (xanthan gum) | 1.0 |
| Formaldehyde (40% aq. solution) | 1.0 |
| Water | to 1 litre |

Analogous suspension concentrate formulations were prepared containing 0.5, 5, 10, 25, 50 and 85% by weight of the compounds of Examples A1-A28, B, C1-C3, D1-D54, E1-E4, F, G1-G3, H1-H7, I1-I2, J, K and L.

HERBICIDAL EXAMPLE A (Pre-Emergence)

Seeds of the weed species listed below were sown in anodised aluminium pans 19 cm long x 9.5 cm wide x 6 cm deep, containing sterilized sandy loam. They were watered and then sprayed with the compounds of the Examples listed below formulated as a solution/suspension in 1:1 by volume of acetone and the wetting agent polyoxyethylene (20 mols) monolaurate solution (2 g per litre).

The concentration of each test compound and volume of application were calculated to give the desired rate of application of the compound in 450 litres per hectare.

After 3 to 4 weeks growth in the controlled environment room (20°C; 75-95% relative humidity; 14 hours per day artificial illumination) the plants were visually assessed for any herbicidal response.

All differences from an untreated control were scored accordingly to an index where 0 = no effect, 1 = 1-24% effect, 2 = 25-69% effect, 3 = 70-89% effect and 4 = 90-100% effect. In the table below, the following letters are used to denote the plant species:

a - Polygonum lapathifolium (Pale persicaria)

b - Galium aparine (cleavers)

c - Chrysanthemum segetum (corn marigold)

d - Alopecurus myosuroides (blackgrass)

e - Elymus repens (Couchgrass)

f - Avena fatua (wild oat)

g - Abutilon theophrasti (velvetleaf)

h - Cyperus rotundus (purple nutsedge)

i - Pharbitis purpurea (morningglory)

j - Echinochloa crus-galli (barnyardgrass)

k - Setaria viridis (green foxtail)

l - Solanum nigrum (black nightshade)

The results obtained were as follows:

| Ex | Kg/ha | a | b | c | d | e | f | g | h | i | j | k | l |
|---|---|---|---|---|---|---|---|---|---|---|---|---|---|
| A3 | 2.5 | 3 | 3 | 3 | 0 | 0 | 1 | 3 | | 3 | 0 | 0 | 4 |
| D1 | 2.5 | 4 | 4 | 4 | 4 | 4 | 3 | 4 | | 4 | 4 | 4 | 4 |

| Ex | Kg/ha | a | b | c | d | e | f | g | h | i | j | k | l |
|---|---|---|---|---|---|---|---|---|---|---|---|---|---|
| D3 | 1.0 | 4 | 4 | 4 | 3 | 4 | 3 | 1 | 2 | 3 | 4 | 4 | 4 |
| D4 | 2.5 | | 4 | 4 | 2 | 2 | 2 | 4 | 3 | 4 | 3 | 3 | 3 |
| D5 | 1.0 | | 3 | 4 | 2 | 3 | 3 | 3 | 0 | 2 | 2 | 2 | 4 |
| D8 | 2.5 | 4 | 2 | 0 | 0 | 3 | 0 | 0 | | 2 | 0 | 0 | |
| D9 | 2.5 | 4 | 4 | 3 | 2 | 3 | 0 | 2 | | 2 | 0 | 2 | |
| D10 | 1.0 | 3 | 4 | 4 | 3 | 3 | 0 | 3 | 4 | 4 | 3 | 4 | |
| D14 | 1.0 | 3 | 4 | 4 | 0 | 0 | 0 | 1 | 0 | 2 | 2 | 3 | |
| D16 | 1.0 | 4 | 4 | 4 | 2 | 3 | 2 | 4 | 0 | 3 | 2 | 3 | |
| D18 | 1.0 | 4 | 4 | 4 | 4 | 4 | 3 | 4 | 3 | 4 | 3 | 4 | |
| D21 | 0.5 | 4 | 4 | 4 | 2 | 3 | 3 | 4 | 4 | 4 | 4 | 4 | 4 |
| D23 | 0.5 | 4 | 4 | 4 | 4 | 4 | 3 | 3 | 2 | 3 | 2 | 4 | 4 |
| D24 | 0.5 | 4 | 4 | 4 | 2 | 2 | 2 | 3 | 3 | 3 | 2 | 3 | 4 |
| D25 | 0.5 | 4 | 4 | 4 | 0 | 3 | 1 | 3 | 4 | 2 | 0 | 0 | 4 |
| D29 | 0.5 | 3 | 4 | 4 | 4 | 4 | 2 | 3 | 3 | 3 | 4 | 4 | 4 |
| D30 | 0.5 | 3 | 4 | 4 | 2 | 2 | 4 | 2 | 2 | 3 | 0 | 0 | 4 |
| D31 | 0.5 | 3 | 4 | 4 | 0 | 0 | 2 | 2 | 0 | 2 | 0 | 0 | 4 |
| D32 | 0.5 | 4 | 4 | 4 | 2 | 2 | 3 | 4 | 2 | 4 | 3 | 2 | 4 |
| D34 | 0.5 | 3 | 4 | 4 | 2 | 2 | 4 | 2 | 4 | 2 | 0 | 0 | 4 |
| G3 | 0.5 | 4 | 3 | 3 | 0 | 0 | 0 | 1 | 0 | 0 | 2 | 3 | 4 |
| H1 | 0.5 | 4 | 4 | 4 | 3 | 4 | 3 | 4 | 4 | 4 | 4 | 4 | 4 |

HERBICIDAL EXAMPLE B (Post-Emergence)

Seeds of the plant species listed below were sown in anodised aluminium pans, 19 cm long x 9.5 cm x 6 cm deep, containing sterilised sandy loam. They were then watered

and placed in a controlled environment room (20°C; 75-95% relative humidity; 14 hours per day artificial illumination). Fourteen or twenty one days after sowing (depending on the species but when most plants had 2 to 3 true leaves) the seedlings received a foliar spray of the compounds of the Examples listed below, formulated as a solution/suspension in 1:1 by volume of acetone and the wetting agent polyoxyethylene (20 mols) monolaurate solution (2 g per litre).

The concentration of each test compound was calculated to give the desired rate of application of the compound in 450 litres per hectare. After 2 to 3 weeks growth in the controlled environment room the plants were visually assessed for any herbicidal response.

All differences from an untreated control were scored according to an index where 0 = no effect, 1 = 1-24% effect, 2 = 25-69% effect, 3 = 70-89% effect and 4 = 90-100% effect. In the table below, the letters used denote the same plant species as in Herbicidal Example A:

The results obtained were as follows:

| Ex | Kg/ha | a | b | c | d | e | f | g | h | i | j | k | l |
|---|---|---|---|---|---|---|---|---|---|---|---|---|---|
| D1 | 2.5 | 4 | 4 | 3 | 3 | | 3 | 4 | | 4 | 4 | 3 | 4 |
| D16 | 1.0 | 3 | 4 | 3 | 3 | 2 | 3 | 4 | 2 | 1 | 2 | 2 | 3 |
| D18 | 1.0 | 2 | 4 | 4 | 3 | 3 | 3 | 2 | 2 | 2 | 2 | 3 | 3 |
| D21 | 0.5 | 2 | 4 | 3 | 2 | 2 | 3 | 2 | 2 | 2 | 2 | 2 | 4 |

| Ex | Kg/ha | a | b | c | d | e | f | g | h | i | j | k | l |
|---|---|---|---|---|---|---|---|---|---|---|---|---|---|
| D23 | 0.5 | 2 | 3 | 2 | 3 | 2 | 3 | 2 | 1 | 1 | 0 | 1 | 4 |
| D29 | 0.5 | 2 | 4 | 2 | 3 | 4 | 2 | 0 | 0 | 2 | 2 | 0 | 2 |
| H1 | 0.5 | 3 | 4 | 3 | 3 | 3 | 3 | 1 | 2 | 2 | 2 | 2 | 2 |

CLAIMS

1. The pyrazolopyrimidine derivatives of the formula:

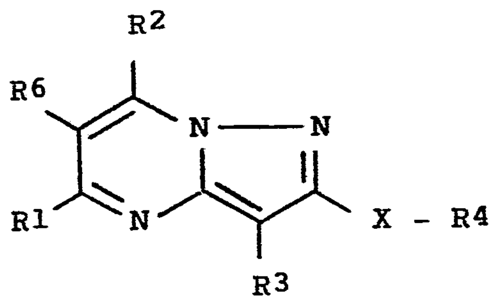

(I)

and the salts thereof, where:

$R^1$ and $R^2$, which may be the same or different, each represent hydrogen, hydroxy, halo, substituted or unsubstituted alkyl, alkoxy, alkylthio, alkenyl, alkynyl or phenyl, or heterocyclyl;

$R^6$ represents hydrogen, halo, cyano, carboxy, alkoxycarbonyl, or sustituted or unsubstituted alkyl or carbamoyl; or $R^6$ and one of $R^1$ and $R^2$ together represent an alkylene chain of 3 or 4 carbon atoms;

$R^3$ represents hydrogen, alkyl, aryl, aralkyl, carboxy, substituted or unsubstituted alkoxycarbonyl, carbamoyl or thiocarbamoyl, cyano or nitro;

X represents $-NR-SO_2-$, $-SO_2-O-$, $-SO_2-NR-$ or $-S(O)_n-CRR'-$, where n is 0, 1 or 2 and R and R' each represent hydrogen, or substituted or unsubstituted alkyl, alkenyl, alkynyl, alkoxycarbonyl, acyl, alkylsulphonyl or arylsuphonyl; and

$R^4$ represents hydrogen, heterocyclyl, or a substituted or unsubstituted alkyl or aryl group.

2. Pyrazolopyrimidine derivatives according to claim 1 wherein $R^1$ represents hydrogen, hydroxy, chloro, alkyl of 1 to 6 carbon atoms optionally substituted by one or more halogen atoms or alkoxy groups of 1 to 4 carbon atoms.

3. Pyrazolopyrimidine derivatives according to claim 1 or claim 2 wherein $R^2$ represents hydrogen, hydroxy, chloro, alkyl of 1 to 6 carbon atoms optionally substituted by one or more halogen atoms or alkoxy groups of 1 to 4 carbon atoms.

4. Pyrazolopyrimidine derivatives according to any of claims 1 to 3 wherein $R^3$ represents carboxy, substituted or unsubstituted alkoxycarbonyl of 2 to 5 carbon atoms, carbamoyl or substituted carbamoyl.

5. Pyrazolopyrimidine derivatives according to any of claims 1 to 4 wherein $R^6$ represents hydrogen, alkyl of 1 to 6 carbon atoms, or chloro.

6. Pyrazolopyrimidine derivatives according to any of claims 1 to 5 wherein X represents $-SO_2NH$.

7. Pyrazolopyrimidine derivatives according to any of claims 1 to 6 wherein $R^4$ represents a phenyl group substituted by one or more chlorine atoms, methyl groups, alkoxycarbonyl groups of 2 to 5 carbon atoms, nitro groups or substituted or unsubstituted amino groups.

8. Pyrazolopyrimidine derivatives according to any of claims 1 to 7 wherein one of $R^1$ and $R^2$ represents methyl and the other represents hydrogen or methyl, $R^6$ is hydrogen, $R^3$ is methoxycarbonyl, ethoxycarbonyl, carbamoyl, methylcarbamoyl or dimethylcarbamoyl, X is $-SO_2NH-$, and $R^4$ is phenyl substituted by one or more fluorine, chlorine or bromine atoms, or methyl or nitro groups.

9. A pyrazolopyrimidine derivative according to any of the preceding claims, which is:
Methyl 2-(2,6-dichlorophenylsulphamoyl)-5,7-dimethylpyrazolo-[1,5-a]pyrimidine-3-carboxylate; or
Methyl 2-(2,6-dichloro-phenylsulphamoyl)-5-methyl-7-trifluoromethylpyrazolo-[1,5-a]pyrimidine-3-carboxylate.

10. A herbicidal composition which comprises from 0.01 to 99% by weight of one or more compounds according to any of claims 1 to 9 in association with a suitable carrier and/or surface active agent.

0787I